# EUROPÄISCHE PATENTSCHRIFT

(11) **EP 2 814 571 B1**
(45) Veröffentlichungstag und Bekanntmachung des Hinweises auf die Patenterteilung: **16.11.2016**
(21) Anmeldenummer: 13711600.0
(22) Anmeldetag: 12.03.2013
(51) Int. Cl.: A61N 5/06, A61F 7/00

(54) **VORRICHTUNG ZUR STIMULATION MIT THERMOREIZEN**
DEVICE FOR STIMULATION BY MEANS OF THERMAL STIMULI
DISPOSITIF DE STIMULATION PAR THERMOEXCITATION

(30) Priorität: 12.03.2012 DE 102012005030
(43) Veröffentlichungstag der Anmeldung: 24.12.2014
(73) Patentinhaber: Forschungszentrum Jülich GmbH, 52425 Jülich (DE)
(72) Erfinder: TASS, Peter Alexander, 52428 Jülich (DE); ROULET, Jean-Christophe, 2523 Lignières (CH); VON BÜREN, Thomas, 3012 Bern (CH); FEHR, Jean-Noel, 2000 Neuchatel (CH); SCHNELL, Urban, 3053 Münchenbuchsee (CH)
(74) Vertreter: Manitz, Finsterwald & Partner GbR
(86) Internationale Anmeldenummer: PCT/EP2013/054984
(87) Internationale Veröffentlichungsnummer: WO 2013/135685

(56) Entgegenhaltungen:
- US-A1- 2005 085 875
- US-A1- 2007 264 455

## Beschreibung

Die Erfindung betrifft eine Vorrichtung zur Stimulation von in der Haut eines Patienten gelegenen Thermorezeptoren mit Thermoreizen.

Für viele Krankheiten, bei denen eine gesteigerte neuronale Synchronisation vorliegt, wie z. B. stark ausgeprägten Funktionsstörungen nach Schlaganfall oder einem Reizdarmsyndrom, gibt es derzeit keine ausreichende Therapie. Kennzeichnend für diese Krankheiten ist, dass infolge der krankhaft übersteigerten Synchronisation im Nervensystem und der damit verbundenen krankhaft verstärkten Vernetzung der betroffenen Nervenzellverbände, gesunde Nervenzelltätigkeit gestört bzw. eingeschränkt ist, und sich stattdessen krankhafte raum-zeitliche Muster der Nervenzelltätigkeit etablieren (z. B. im Rahmen einer Maladaption).

Bei diesen Erkrankungen sind umschriebene Nervenzellverbände, z. B. im Gehirn oder in enteralen Ganglien, krankhaft, typischerweise übersteigert synchron, aktiv. D. h., eine große Anzahl von Neuronen bildet synchron Aktionspotentiale aus; die beteiligten Neuronen feuern übermäßig synchron. Beim Gesunden hingegen feuern die Neuronen in diesen Hirngebieten qualitativ anders, z. B. auf unkorrelierte Weise.

Das Dokument US 2005 / 0 085 875 A1 offenbart eine modular aufgebaute Vorrichtung zur Stimulation mit Thermoreizen.

Der Erfindung liegt die Aufgabe zugrunde, eine Vorrichtung zur Stimulation mit Thermoreizen anzugeben, mit der sich eine gegenüber dem Stand der Technik effizientere Desynchronisation und ein lang anhaltendes Verlernen pathologischer synaptischer Vernetzung erzielen lässt.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur Stimulation von in der Haut eines Patienten gelegenen Thermorezeptoren mit Thermoreizen während des Betriebs;
- Fig. 2: eine Darstellung des Absorptionskoeffizienten für Wasser;
- Fig. 3: eine Darstellung des errechneten Prozentsatzes der absorbierten Strahlung gegen die Wellenlänge für unterschiedliche Absorptionstiefen;
- Fig. 4A bis 4D: schematische Darstellungen verschiedener Ausgestaltungen einer Stimulationseinheit zur Erzeugung elektromagnetischer Strahlung mit unterschiedlichen Wellenlängen;
- Fig. 5A bis 5F: schematische Darstellungen verschiedener Ausgestaltungen einer Stimulationseinheit zur Formung der emittierten elektromagnetischen Strahlung;
- Fig. 6A bis 6I: schematische Darstellungen verschiedener optischer Elemente zur Erzeugung homogen verteilter elektromagnetischer Strahlung;
- Fig. 7A bis 7C: schematische Darstellungen einer Ausgestaltung einer Stimulationseinheit zur Erzeugung von Thermoreizen;
- Fig. 8 bis 10: schematische Darstellungen von Ausgestaltungen einer Vorrichtung zur Thermostimulation mit einem oder mehreren Lasern oder anderen Wärmestrahlern zur Erzeugung von elektromagnetischer Strahlung;
- Fig. 11A und 11B: schematische Darstellungen einer Ausgestaltung einer Vorrichtung zur Thermostimulation mit mehreren organischen Leuchtdioden;
- Fig. 12A bis 13B: schematische Darstellungen von Ausgestaltungen einer Vorrichtung zur Thermostimulation mit mechanisch und elektrisch miteinander verbindbaren Stimulationseinheiten;
- Fig. 14: eine schematische Darstellung eines Thermoreizes;
- Fig. 15: eine schematische Darstellung einer CR-Stimulation mittels Thermoreizen; und
- Fig. 16 und 17: schematische Darstellungen von weiteren CR-Stimulationen mittels Thermoreizen.

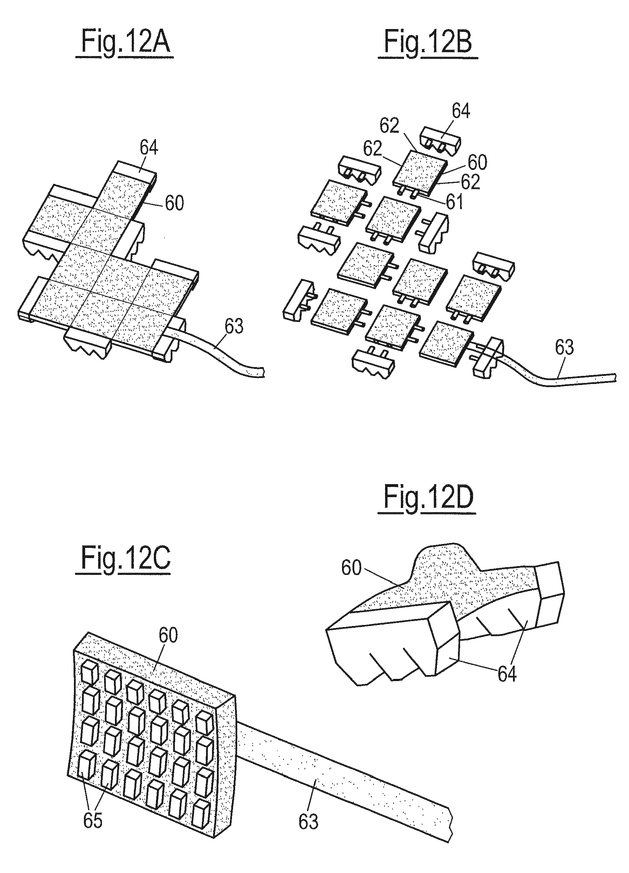

Es sei darauf hingewiesen, dass nur die Fig. 12A bis 12D Ausführungsformen der erfindungsgemäßen Vorrichtung zeigen.

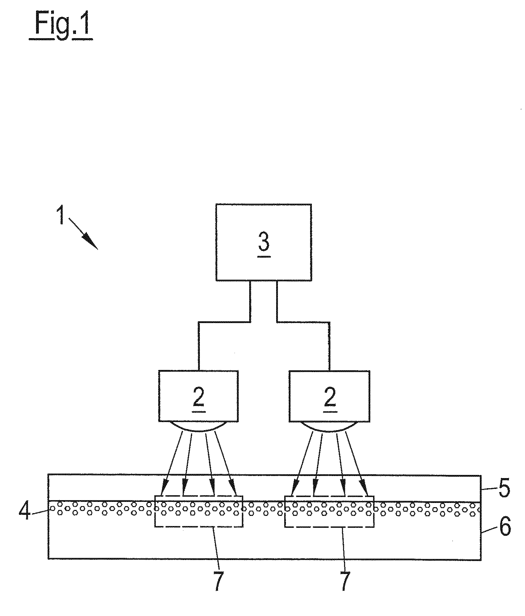

Fig. 1 zeigt schematisch eine Vorrichtung 1 zur Stimulation von in der Haut eines Patienten gelegenen Thermorezeptoren mit Thermoreizen. Insbesondere handelt es sich bei der Vorrichtung 1 um einen photodynamischen Stimulator. Die Vorrichtung 1 enthält eine Mehrzahl von Stimulationseinheiten 2 zur Bestrahlung der Haut des Patienten mit elektromagnetischer Strahlung. Der Wellenlängenbereich der von den Stimulationseinheiten 2 emittierten elektromagnetischen Strahlung ist einstellbar. Ferner ist eine Steuereinheit 3 zur Steuerung der Stimulationseinheiten 2 vorgesehen. Die Stimulationseinheiten 2 und insbesondere auch die Steuereinheit 3 sind nicht-invasive Einheiten, d. h., während des Betriebs der Vorrichtung 1 befinden sie sich außerhalb des Körpers des Patienten und werden nicht operativ in den Körper des Patienten implantiert. Die Mehrzahl von Stimulationseinheiten 2 ermöglicht es, unterschiedliche rezeptive Bereiche der Haut über die einzelnen Stimulationseinheiten 2 zeitlich und räumlich koordiniert zu stimulieren.

Die von den Stimulationseinheiten 2 emittierte elektromagnetische Strahlung wird in der Haut des Patienten absorbiert. Durch die Absorption der elektromagnetischen Strahlung werden Bereiche der Haut erwärmt und idealerweise werden dadurch in der Haut gelegene Thermorezeptoren 4 mit Thermoreizen stimuliert. Die Thermorezeptoren 4 befinden sich - wie in Fig. 1 schematisch gezeigt - direkt unter der Epidermis 5 und der dermalen Papille in der Dermis 6 (typischerweise in einer Tiefe von ca. 150 µm unterhalb der Hautoberfläche). Vorzugsweise wird die Wellenlänge der von den Stimulationseinheiten 2 emittierten elektromagnetischen Strahlung derart eingestellt, dass die elektromagnetische Strahlung von einem Bereich 7 der Dermis 6, in dem sich die Thermorezeptoren 4 befinden, und nicht oder nur wenig von der Epidermis 5 absorbiert wird.

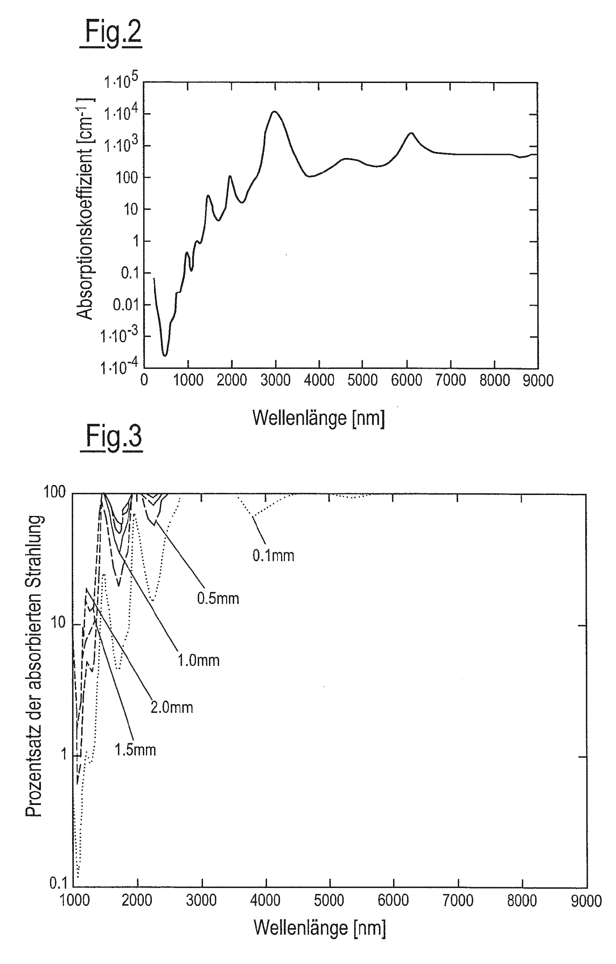

Für die Thermostimulation ist Infrarotstrahlung mit Wellenlängen im Bereich von 780 nm bis 1 mm (und insbesondere im nahen Infrarotbereich von 780 nm bis 3.000 nm) die geeignetste elektromagnetische Strahlung, da sie von der Dermis stark absorbiert wird. Oberhalb einer Wellenlänge von 1.000 nm ist für die Absorption der Strahlung in der Dermis in erster Linie das im Blut enthaltene Wasser verantwortlich. Wie die Darstellung des Absorptionskoeffizienten für Wasser in Fig. 2 zeigt, weist Wasser eine hohe Absorptionsfähigkeit für Strahlung mit Wellenlängen von über 1.000 nm auf. Da die Epidermis keine Blutgefäße enthält, ist die Wasserkonzentration dort niedriger als in der Dermis. Dies ermöglicht es, durch die Wahl eines geeigneten Wellenlängenspektrums, eine Erwärmung der Dermis bei gleichzeitig nur geringer Erwärmung der Epidermis zu erzielen.

Ultraviolettstrahlung ist für die Thermostimulation eher ungeeignet, da durch diese hochenergetische Strahlung die Haut leicht geschädigt werden kann. Außerdem wird Ultraviolettstrahlung von in der Epidermis enthaltenem Melanin stark absorbiert. Das für den Menschen sichtbare Licht weist diese Nachteile nicht auf, wird jedoch vergleichsweise wenig in der Epidermis und der Dermis absorbiert.

Bei der Verwendung von Infrarotstrahlung für die Erzeugung von Thermoreizen ist zu beachten, dass auch in der Epidermis Wasser vorhanden ist und die Strahlung demnach auch dort absorbiert wird. Es ist daher erforderlich, das richtige Maß zu finden zwischen einem hohen Absorptionskoeffizienten, der zu einer starken Absorption der Strahlung in einer vergleichsweise dünnen Schicht bei jedoch nur geringer Eindringtiefe der Strahlung führt, und einem niedrigeren Absorptionskoeffizienten, der ein tieferes Eindringen der Strahlung ermöglicht (und somit eine Absorption in der Dermis), aber auch zu einer Absorption der Strahlung in einer dickeren Schicht führt. Zur Veranschaulichung dieses Zusammenhangs ist in Fig. 3 der errechnete Prozentsatz der absorbierten Strahlung gegen die Wellenlänge für unterschiedliche Absorptionstiefen aufgetragen (wobei davon ausgegangen wird, dass für die Reizung der Thermorezeptoren der Absorptionsprozess im Wasser maßgeblich ist).

Es ist vorgesehen, dass die Wellenlänge der applizierten elektromagnetischen Strahlung derart eingestellt wird, dass die in der Dermis gelegenen Thermorezeptoren optimal stimuliert werden. Da das Absorptionsverhalten der Haut individuell unterschiedlich ist, wird vorteilhafterweise für jeden Patienten vor der Therapie die Wellenlänge bzw. der Wellenlängenbereich ermittelt, mit der bzw. dem sich bestmögliche Stimulationsergebnisse erzielen lassen.

Als Strahlungsquellen zur Erzeugung der elektromagnetischen Strahlung können dem Fachmann bekannte Infrarotstrahlungsquellen eingesetzt werden. Beispiele hierfür sind Leuchtdioden (LEDs), organische Leuchtdioden (OLEDs), Superlumineszenz-Leuchtdioden (SLEDs), Halbleiter-Laserdioden, wie z. B. DH (Double Heterostructure)-Laser, Quantentopf-Laser (Quantum Well Laser), Quanten-Kaskaden-Laser (Quantum Cascade Laser), SCH (Separate Confinement Heterostructure)-Laser, DFB (Distributed Feedback)-Laser und VCSEL (Vertical Cavity Surface-Emitting)-Laser, sowie Gaslaser und Festkörperlaser. Als Strahlungsquellen, um die Thermorezeptoren in einer Tiefe von ca. 100 bis 200 µm unterhalb der Hautoberfläche und insbesondere in einem Bereich um 150 µm stimulieren zu können, sind insbesondere Ho:YAG (Holmium:YAG)-Laser mit einer Wellenlänge von 2,1 µm und GaAs-Laserdioden im Wellenlängenbereich von 1,3 bis 1,5 µm geeignet.

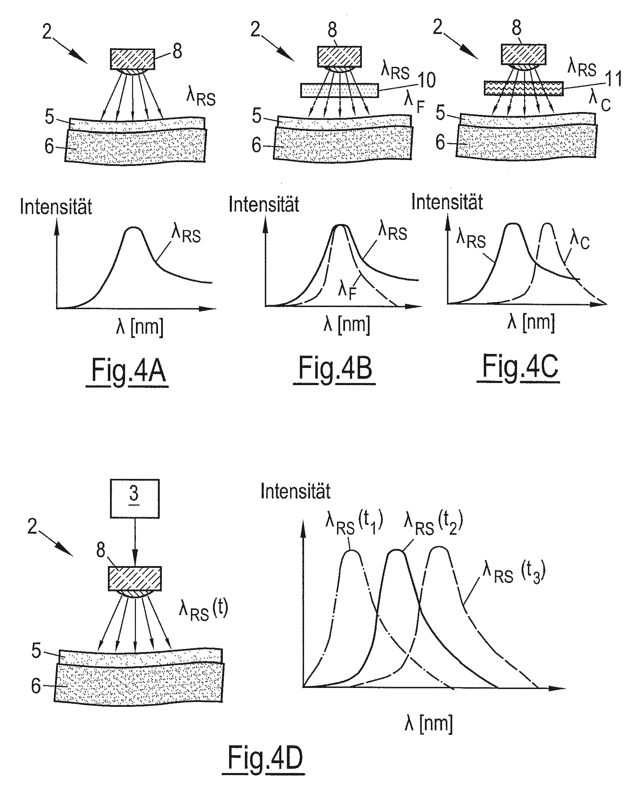

Fig. 4A bis 4D zeigen schematisch verschiedene Ausgestaltungen einer Stimulationseinheit 2, mit der sich Strahlung eines gewünschten Spektralbereichs erzeugen lässt, um so die in der Haut des Patienten gelegenen Thermorezeptoren optimal stimulieren zu können.

Die in Fig. 4A gezeigte Strahlungsquelle 8 erzeugt elektromagnetische Strahlung mit der Wellenlängenverteilung λ_{RS}.

In Fig. 4B durchläuft die von der Strahlungsquelle 8 emittierte Strahlung ein optisches Filter 10, wodurch bestimmte Anteile des Spektrums eliminiert werden und sich am Ausgang des optischen Filters 10 eine Wellenlängenverteilung λ_{F} ergibt. Es können verschiedene optische Filter 10 vorgesehen sein, um Strahlung mit unterschiedlichen Wellenlängenverteilungen λ_{F} erzeugen zu können. Die Stimulationseinheit 2 kann so ausgestaltet sein, dass wahlweise eines der optischen Filter 10 (oder auch keines der optischen Filter 10) vor die Strahlungsquelle 8 geschaltet wird, um dadurch den für die Stimulation der Thermorezeptoren optimalen Wellenlängenbereich zu erzeugen.

Fig. 4C zeigt ein fluoreszierendes Material 11, mit dem das von der Strahlungsquelle 8 erzeugte Spektrum verschoben wird und die in Fig. 4C gezeigte Wellenlängenverteilung λ_{C} erhalten wird. Es können verschiedene fluoreszierende Materialien 11 bereitgestellt werden, die wahlweise vor die Strahlungsquelle 8 geschaltet werden können, um unterschiedliche Wellenlängenverteilungen λ_{C} zu erzeugen.

Fig. 4D zeigt eine Ausgestaltung der Stimulationseinheit 2 mit einer Strahlungsquelle 8, deren Strahlungsspektrum von der Steuereinheit 3 einstellbar ist. Beispielsweise kann die Strahlungsquelle 8 ein durchstimmbarer Laser sein. In Fig. 4D sind beispielhaft drei unterschiedliche Wellenlängenverteilungen λ_{RS}(t₁), λ_{RS}(t₂) und λ_{RS}(t₃) dargestellt. Durch eine geeignete Einstellung der Strahlungsquelle 8 lässt sich die Lage und Ausdehnung des in Fig. 1 gezeigten Bereichs 7 in der Dermis, in dem eine Absorption der Strahlung und damit eine Erwärmung des Gewebes bewirkt wird, einstellen.

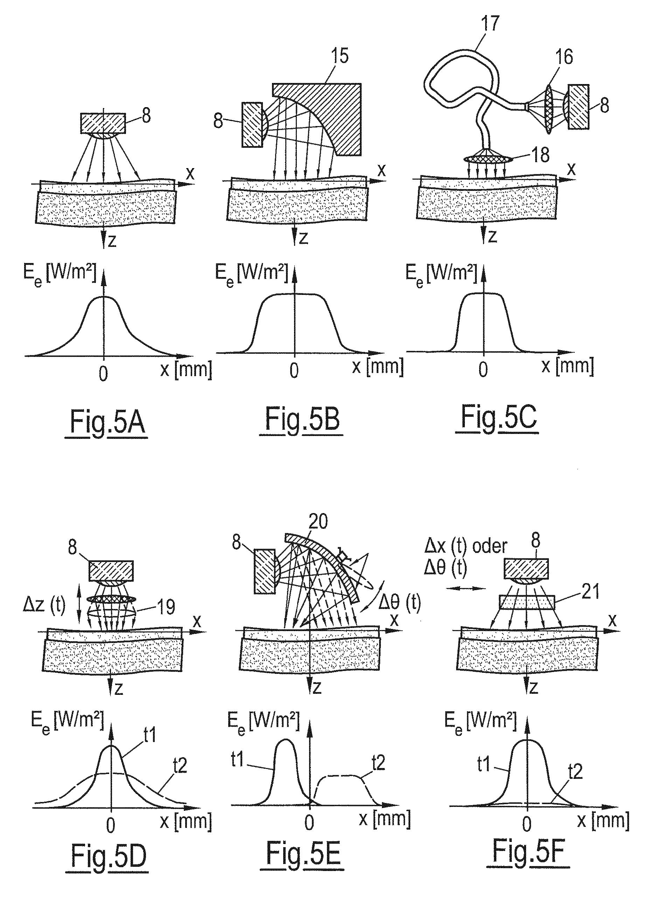

Die von der Strahlungsquelle 8 emittierte Strahlung kann durch die Verwendung entsprechender optischer Elemente geformt werden, wie im Folgenden beispielhaft anhand der Fig. 5A bis 5F erläutert wird. In den Fig. 5A bis 5F ist neben den verschiedenen optischen Elementen auch die damit erzeugte Strahlungsleistung Eₑ an der Hautoberfläche dargestellt.

Während Fig. 5A die Strahlungsquelle 8 ohne optische Elemente zur Strahlformung zeigt, ist in Fig. 5B im Strahlengang der Strahlungsquelle 8 ein Spiegel 15 angeordnet, der gleichzeitig als Linse wirkt.

In Fig. 5C wird die Strahlung der Strahlungsquelle 8 mittels einer Linse 16 in einen Lichtleiter 17 eingekoppelt. Die von dem Lichtleiter 17 ausgegebene Strahlung durchläuft eine weitere Linse 18, die eine über einen gewissen Hautbereich homogen verteilte Strahlungsleistung erzeugt.

Während die Positionen der in den Fig. 5B und 5C gezeigten optischen Elemente fest sind, lassen sich die Positionen der in den Fig. 5D bis 5F gezeigten optischen Elemente variieren.

In Fig. 5D ist eine konvexe Linse 19 gezeigt, die sich entlang der z-Achse verschieben lässt. Die dadurch erzeugte Strahlungsleistung Eₑ an der Hautoberfläche ist für zwei Positionen der Linse 19 dargestellt.

Fig. 5E zeigt ein verkipp- und deformierbares Spiegelsystem 20, das eine Variation sowohl des Bestrahlungsorts als auch der Strahlungsleistung zulässt.

Fig. 5F zeigt einen mechanischen Verschluss 21, der z. B. als Blende, rotierender Verschluss, Schieber oder dergleichen ausgestaltet sein kann und die von der Strahlungsquelle 8 erzeugte Strahlung entweder vollständig oder aber gar nicht durchlässt.

Es ist denkbar, ein Kühlsystem, wie beispielsweise einen Ventilator oder einen thermo-elektrischen Kühler, zur Kühlung der Epidermis einzusetzen, um dadurch die Wirksamkeit der Thermostimulation zu erhöhen. Da die Thermorezeptoren in erster Linie auf Temperaturgradienten ansprechen, kann es vorteilhaft sein, zwischen aufeinanderfolgenden Stimulationsabschnitten die durch die Thermoreize in das Stimulationsgebiet eingebrachte Wärme aus dem Stimulationsgebiet wieder zu entfernen.

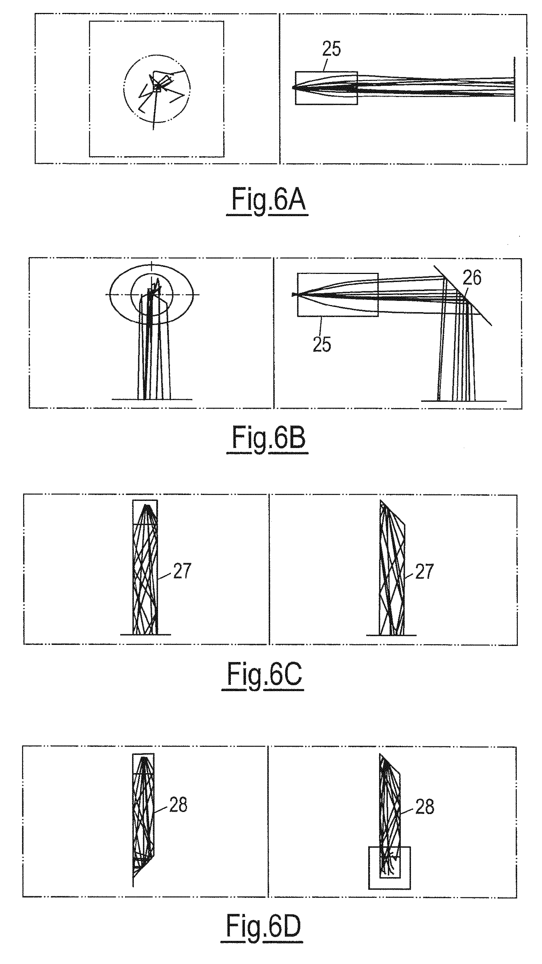

Um Verletzungen des Patienten durch lokal überhöhte Strahlungsleistungen auszuschließen, ist es vorteilhaft, die Strahlung derart zu formen, dass sie über eine bestimmte Fläche verteilt ist und an keiner Stelle ein vorgegebener Grenzwert der Strahlungsleistung überschritten wird. Idealerweise ist die Strahlungsleistung auf der Haut des Patienten über einen gewissen Bereich weitgehend homogen verteilt. Zu diesem Zweck können beispielsweise die in den Fig. 6A bis 6H gezeigten optischen Elemente verwendet werden, wobei die optischen Elemente auf der linken und rechten Seite der Figuren aus jeweils unterschiedlichen Blickrichtungen dargestellt sind.

Fig. 6A und 6B zeigen eine Gradientenlinse 25 (auch GRIN- oder Gradienten-Index-Linsen genannt), die die von einer Strahlungsquelle erzeugte Strahlung kollimiert. In Fig. 6B ist zusätzlich ein Spiegel 26 in dem Strahlengang angeordnet, mit dem die Strahlung auf das Zielgebiet gerichtet wird. Durch die Verwendung des Spiegels 26 kann die Stimulationseinheit vergleichsweise flach ausgeführt werden.

Fig. 6C und 6D zeigen Lichtleiter 27, 28 von jeweils zwei verschiedenen Seiten. Beide Lichtleiter 27, 28 haben einen quadratischen Querschnitt, jedoch können ihre Querschnitte auch andere geometrische Formen aufweisen. Ferner verfügen die Lichtleiter 27, 28 an einem Ende über eine um 45° abgeschrägte Fläche, an welcher die Strahlungsquelle befestigt werden kann. Am anderen Ende der Lichtleiter 27, 28 tritt die Strahlung mit einer sehr präzisen und homogenen Verteilung aus. Dieses Ende kann in Kontakt mit der Haut des Patienten gebracht werden. Der Lichtleiter 28 verfügt zudem über eine abgeschrägte Fläche am unteren Ende, welche bewirkt, dass die Strahlung seitlich aus dem Lichtleiter 28 austritt.

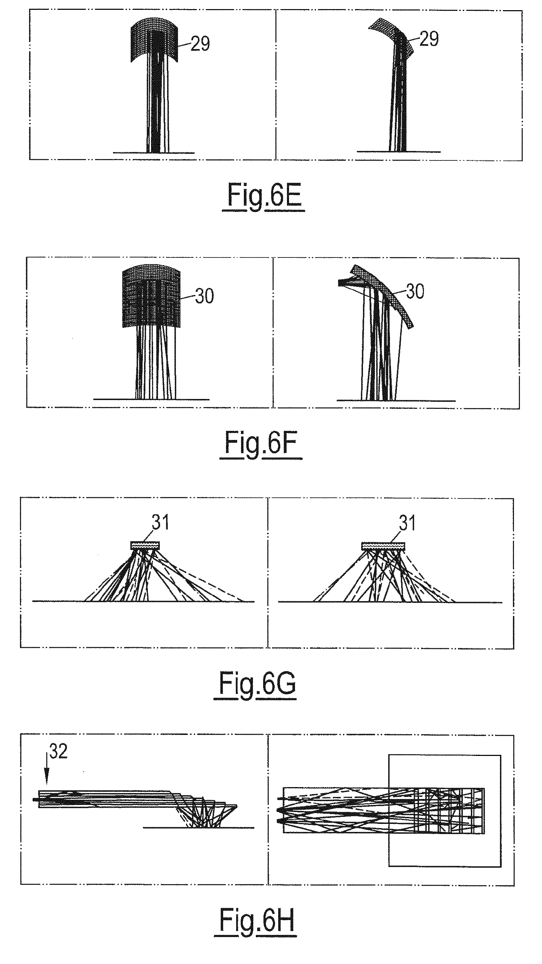

Fig. 6E und 6F zeigen ein bzw. drei toroidale Spiegel 29, 30 mit unterschiedlichen Krümmungsradien in Richtung der x- und y-Achse, wodurch sich eine präzise Strahlformung erzielen lässt.

Fig. 6G zeigt ein optisches Element 31 mit einer reflektierenden Oberseite sowie reflektierenden Seitenflächen. Die Unterseite des optischen Elements 31 streut die Strahlung in Richtung des Zielorts. Die streuende Unterseite kann eine Gauß-Verteilung der Strahlung bewirken.

Fig. 6H zeigt eine Anordnung 32 mit einer Vielzahl von optischen Elementen, die jeweils unterschiedliche Längen und Winkel aufweisen. Die Anordnung 32 erlaubt die Erzeugung eines komplexen Strahlungsverlaufs.

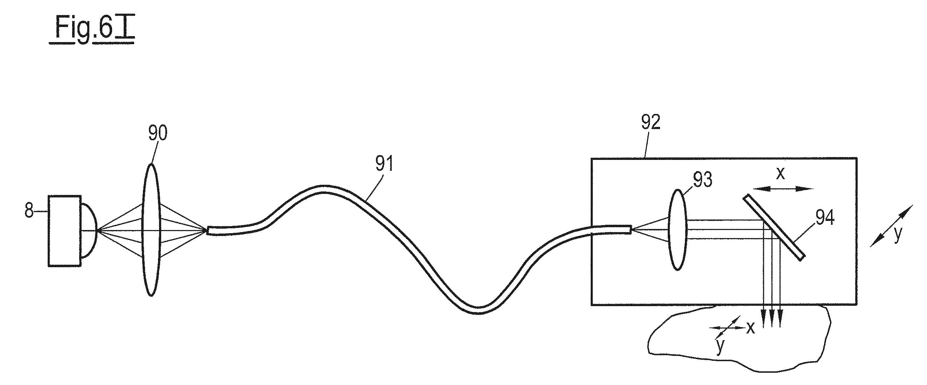

Fig. 6I zeigt eine Vorrichtung mit einer Strahlungsquelle 8 zur Erzeugung von elektromagnetischer Strahlung, die mittels einer Linse 90 in einen Lichtleiter 91 eingekoppelt wird. Der Lichtleiter 91 führt die elektromagnetische Strahlung zu einem Scanner 92, in dem sich eine Linse 93 und ein in x- und y-Richtung verstellbarer Spiegel 94 oder ein anderes entsprechend verstellbares Element befinden. Durch die Verstellung der Position des Spiegels 94 kann gezielt eine bestimmte Hautstelle stimuliert werden. Ferner können verschiedene Stellen eines bestimmten Bereichs der Haut sukzessive stimuliert werden.

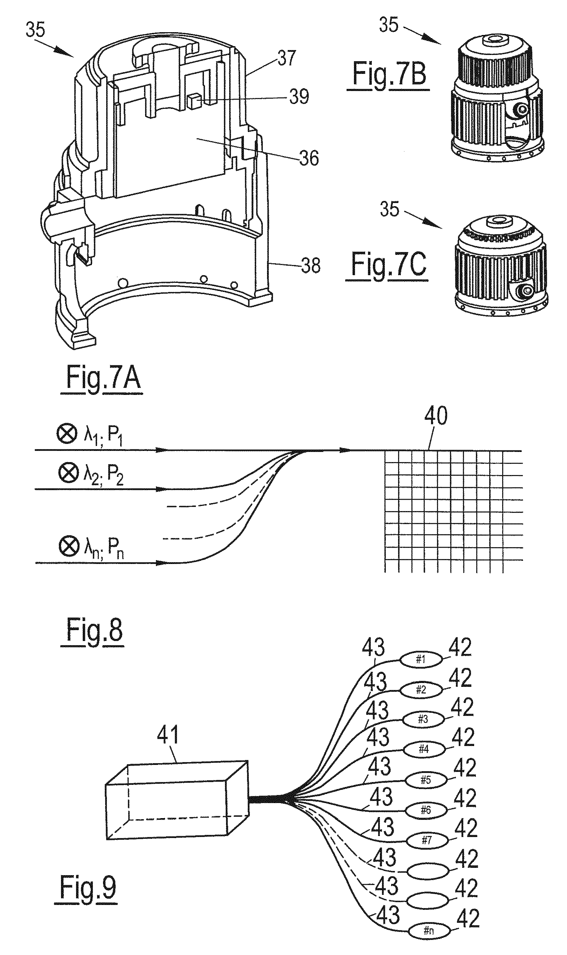

Fig. 7A zeigt eine Stimulationseinheit 35 zur Erzeugung von Thermoreizen mittels elektromagnetischer Strahlung in einer Aufschnitt-Ansicht. In den Fig. 7B und 7C ist die Stimulationseinheit 35 in zwei verschiedenen Betriebszuständen gezeigt. Eine in die Stimulationseinheit 35 integrierte filamentartige Infrarotstrahlungsquelle 36 dient zur Erzeugung der elektromagnetischen Strahlung. Die Infrarotstrahlungsquelle 36 ist in ein zylinderförmiges Gehäuse 37 eingebracht, welches über ein Gewinde mit einem zylinderförmigen Abstandshalter 38 verbunden ist. Zur Behandlung eines Patienten wird die Stimulationseinheit 35 mit der Unterseite des Abstandshalters 38 auf die Haut des Patienten aufgesetzt. Der Abstand zwischen der Strahlungsquelle 36 und der Haut des Patienten lässt sich verstellen (Fig. 7B und 7C zeigen zwei unterschiedliche Abstände). Ferner gewährleistet der Abstandshalter 38 einen Minimalabstand der Strahlungsquelle 36 von der Haut des Patienten, um eine Gefährdung des Patienten durch zu hohe Strahlungsleistungen auszuschließen. Zur Kühlung enthält die Stimulationseinheit 35 passive Kühlelemente. Als weiteres Sicherheitselement verfügt die Stimulationseinheit 35 über einen Temperatursensor 39, der es erlaubt, eine eventuelle Überhitzung der Stimulationseinheit 35 festzustellen.

Die Fig. 8 bis 10 zeigen Ausgestaltungen von Stimulationseinheiten mit einer oder mehreren gemeinsam genutzten Strahlungsquellen, z. B. einem oder mehreren Lasern. Die von den Strahlungsquellen emittierte Strahlung wird dabei über Lichtleiter zu den Stimulationseinheiten geführt.

Fig. 8 zeigt eine Ausgestaltung mit n Strahlungsquellen, die jeweils eine Strahlung der Wellenlänge λᵢ und der Strahlungsleistung Pi erzeugen (mit i = 1,..., n). Die verschiedenen Strahlungen werden in einen Lichtleiter eingekoppelt und zu einer auf der Haut des Patienten befestigten Stimulationsmatrix 40 geführt. Die unterschiedlichen Wellenlängen λᵢ weisen unterschiedliche Absorptionskoeffizienten auf und können daher unterschiedlich tief unter der Hautoberfläche gelegene Bereiche der Dermis stimulieren.

Fig. 9 zeigt einen Demultiplexer 41, wie er beispielsweise aus der Telekommunikationstechnik bekannt ist, in den die Strahlung einer Strahlungsquelle eingekoppelt wird und der n Stimulationseinheiten 42 speist. In den Demultiplexer 41 können beispielsweise ein oder mehrere verstellbare Spiegel integriert sein, die den von der Strahlungsquelle erzeugten Strahl auf n Ausgänge des Demultiplexers 41 verteilen, von wo aus die Strahlung mit Hilfe von Lichtleitern 43 zu den Stimulationseinheiten 42 geführt wird. Der Demultiplexer 41 ermöglicht eine individuelle Ansteuerung jeder Stimulationseinheit 42.

Weiterhin kann vorgesehen sein, dass die Strahlung von M Strahlungsquellen in den Demultiplexer 41 eingekoppelt wird. Die M Strahlungsquellen können Strahlung mit unterschiedlichen Wellenlängen bzw. Wellenlängenbereichen oder auch mit überlappenden Wellenlängenbereichen erzeugen.

Eine weitere Lösung, um die Strahlung an eine Vielzahl von Stimulationseinheiten zu verteilen, kann darin bestehen, in jede Stimulationseinheit einen Demultiplexer zu integrieren, der die Strahlung sowohl zur Haut des Patienten als auch zur jeweils benachbarten Stimulationseinheit leitet.

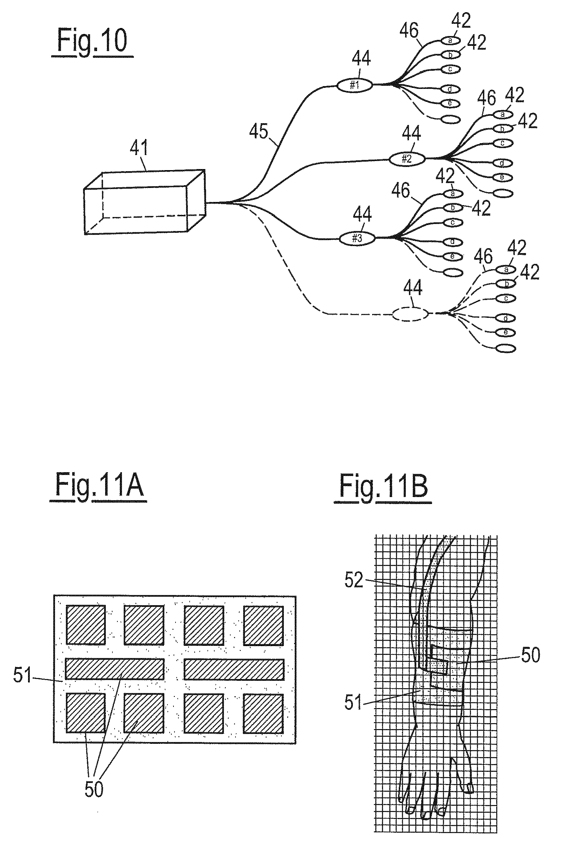

Ferner ist eine Hybridlösung, wie sie beispielhaft in Fig. 10 gezeigt ist, denkbar. Dort sind Splitter 44 zwischen den Demultiplexer 41 und die Stimulationseinheiten 42 geschaltet. Der Demultiplexer 41 verteilt die Infrarotstrahlung über Leitungen 45 an die Splitter 44 und versorgt die Splitter 44 zudem über die Leitungen 45 mit einer Versorgungsspannung. Die Splitter 44 leiten die Strahlung über Lichtleiter 46 an die einzelnen Stimulationseinheiten 42 weiter.

Fig. 11A und 11B zeigen schematisch eine Vorrichtung zur Stimulation mit Thermoreizen, in welcher die Stimulationseinheiten als organische Leuchtdioden (OLEDs) 50 ausgeführt sind. Die organischen Leuchtdioden 50 haben eine geeignete Größe und sind auf einen flexiblen Träger 51 aufgebracht, der - wie Fig. 11B zeigt - beispielsweise an einen Arm oder einem Bein oder einem sonstigen Körperteil des Patienten befestigt werden kann. Über ein oder mehrere Versorgungsleitungen 52 sind die organischen Leuchtdioden 50 mit einer Steuereinheit verbunden. Jede Leuchtdiode 50 kann individuell angesteuert werden.

Fig. 12A bis 12D zeigen schematisch eine weitere Vorrichtung zur Stimulation mit Thermoreizen. Die Vorrichtung ist aus einer Vielzahl von Stimulationseinheiten 60 zusammengesetzt, die jeweils an einer Hauptoberfläche elektromagnetische Strahlung emittieren. Die Stimulationseinheiten 60 sind identisch aufgebaut und weisen jeweils ein rechteckiges, insbesondere quadratisches Umfangsprofil auf. An einer Seitenfläche weist jede Stimulationseinheit 60 einen Stecker 61, an den drei übrigen Seitenflächen entsprechende Buchsen 62 auf. Zwei Stimulationseinheiten 60 können mechanisch und elektrisch miteinander verbunden werden, indem der Stecker 61 der einen Stimulationseinheit 60 in eine Buchse 62 der anderen Stimulationseinheit 60 eingeführt wird. Auf diese Art und Weise können beliebig viele Stimulationseinheiten 60 miteinander verbunden und den jeweiligen anatomischen Gegebenheiten des Patienten angepasst werden. Eine der Stimulationseinheiten 60 weist ferner ein Verbindungskabel 63 zur Verbindung mit einer Steuereinheit auf. Es kann vorgesehen sein, dass diese Stimulationseinheit 60 keinen Stecker 61 aufweist, sondern an allen vier Seitenflächen dieser Stimulationseinheit Buchsen 62 angeordnet sind.

Anstelle eines viereckigen, insbesondere rechteckigen oder quadratischen Umfangsprofils können die Stimulationseinheiten 60 auch die Form eines anderen, insbesondere regelmäßigen Polygons haben, wie z. B. eines Dreiecks, Fünfecks, Sechsecks oder dergleichen.

Die nicht an benachbarte Stimulationseinheiten 60 angrenzenden Seitenflächen der Stimulationseinheiten 60 können mit Abstandshaltern 64 belegt werden. Diese sind beispielsweise aus Kunststoff oder einem anderen elektrisch isolierenden Material gefertigt. Eine Seitenfläche der Abstandshalter 64 ist jeweils in der Form eines Steckers ausgebildet und kann damit an der Buchse 62 einer Stimulationseinheit 60 befestigt werden. Die Abstandshalter 64 schaffen einen definierten Abstand zwischen den Unterseiten der Stimulationseinheiten 60 und der Haut des Patienten, auf welche die Stimulationseinheiten 60 aufgebracht werden, und begrenzen dadurch passiv die maximale Leistungsdichte (Vermeidung von Hautverbrennungen).

An der Unterseite jeder Stimulationseinheit 60 ist eine Vielzahl von Leuchtdioden 65 als Strahlungsquellen angeordnet. Die Leuchtdioden 65 emittieren während des Betriebs insbesondere Infrarotstrahlung. Gemäß einer Ausgestaltung kann jede der Leuchtdioden 65 individuell angesteuert werden. Alternativ kann auch vorgesehen sein, dass die Leuchtdioden 65 in unterschiedlichen Gruppen zusammengefasst werden und die Leuchtdioden 65 jeweils einer Gruppe die gleichen Steuersignale empfangen.

Jede Stimulationseinheit 60 ist elektrisch über die jeweils dazwischen geschalteten Stimulationseinheiten 60 mit der Steuereinheit verbunden. Die Steuereinheit versorgt die Stimulationseinheiten 60 neben den Steuersignalen mit einer Versorgungsspannung. Alternativ kann die Steuerung auch vollständig oder zumindest teilweise in die Stimulationseinheiten 60 integriert sein. Ferner ist es denkbar, dass die Steuereinheit die äußere Form einer Stimulationseinheit 60 (oder zumindest eine ähnliche Form) aufweist und über eine Steckverbindung mit den Stimulationseinheiten 60 verbunden ist, d. h., in diesem Fall ist die Steuereinheit während des Betriebs zusammen mit den Stimulationseinheiten 60 auf der Haut des Patienten befestigt.

Das modulare Konzept der in Fig. 12A und 12B dargestellten Stimulationsvorrichtung erlaubt es, eine beliebige Fläche auf der Haut des Patienten mit Stimulationseinheiten 60 zu bedecken.

Ferner kann vorgesehen sein, dass Stimulationseinheiten 60 mit unterschiedlichen Strahlungsspektren bereitgestellt werden. Vor Beginn der Therapie können dann für jeden Patienten die Stimulationseinheiten 60 mit dem optimalen Strahlungsspektrum ausgewählt werden. Weiterhin ist es denkbar, dass ein Patient aufgrund einer unterschiedlichen Beschaffenheit der Haut an verschiedenen Stellen der Haut mit Stimulationseinheiten therapiert wird, die Strahlung unterschiedlicher Wellenlängenbereiche emittieren.

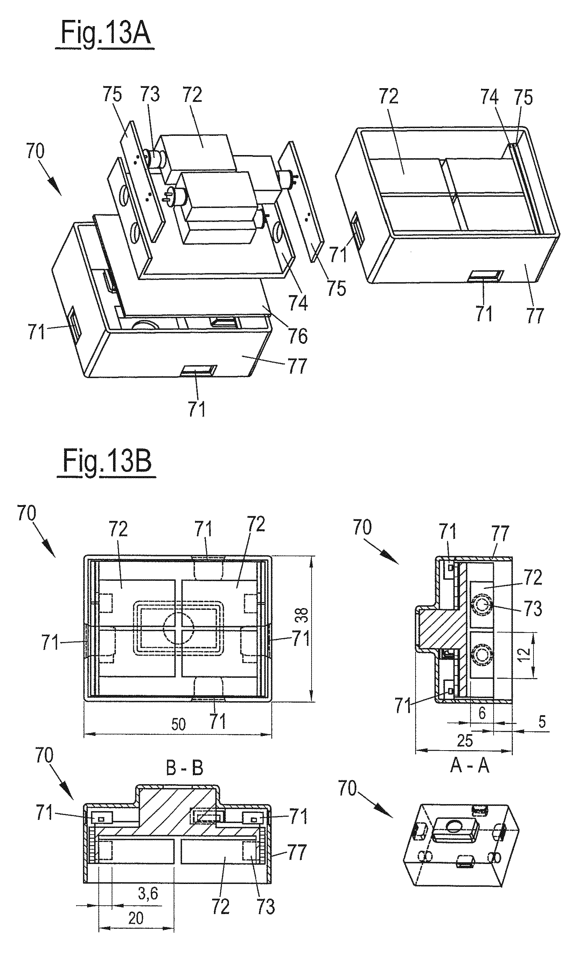

Fig. 13A und 13B zeigen eine Stimulationseinheit 70 als eine Variante der Stimulationseinheit 60 aus Fig. 12A bis 12D. Die Stimulationseinheit 70 weist ein rechteckförmiges Umfangsprofil auf, wobei an jeder Seitenfläche der Stimulationseinheit 70 eine Buchse 71 angeordnet ist.

In der Stimulationseinheit 70 befinden sich vier Strahlung streuende Platten 72. In eine entsprechende Aussparung jeder Platte 72 ist eine Leuchtdiode 73 integriert. Bis auf die Unterseiten der Platten 72 können alle anderen Flächen der Platten 72 reflektierend sein. Die Unterseiten der Platten 72 sind für die von den Leuchtdioden 73 erzeugte Strahlung durchlässig.

Die Platten 72 sind zusammen mit den Leuchtdioden 73 in einem Metallgehäuse 74 untergebracht, das auch als Wärmesenke dient. Ferner sind jeweils zwei Leuchtdioden 73 auf eine Elektronikplatine 75 montiert. Das Metallgehäuse 74 weist Bohrungen auf, durch welche die Leuchtdioden 73 in die Aussparungen der jeweiligen Platten 72 geschoben werden können. Darüber hinaus ist eine weitere Elektronikplatine 76 vorgesehen, auf die Bauelemente zur Steuerung der Leuchtdioden 73 und die Buchsen 71 montiert sind.

Alle vorstehend beschriebenen Bauelemente sind in ein Gehäuse 77 eingefügt, das nach unten offen ist. Das Gehäuse 77 ist während des Betriebs der Stimulationseinheit 70 mit seiner Unterseite auf der Haut des Patienten befestigt. Aufgrund der Form des Gehäuses 77 und der Anordnung der Platten 72 in dem Gehäuse 77 wird ein definierter Abstand zwischen den Platten 72 und der Haut des Patienten geschaffen.

Verschiedene Schnitte durch die Stimulationseinheit 70 sowie eine perspektivische Ansicht der Stimulationseinheit 70 sind in Fig. 13B gezeigt. Dort sind beispielhafte Abmessungen in Millimetern angegeben.

Die von den Leuchtdioden 73 erzeugte Strahlung, bei der es sich insbesondere um Infrarotstrahlung in einem geeigneten Wellenlängenbereich handeln kann, wird von den Platten 72 diffus gestreut. Die Unterseiten der Platten 72 stellen homogene Abstrahlflächen dar.

Mehrere Stimulationseinheiten 70 können genauso wie die in Fig. 12A und 12B gezeigten Stimulationseinheiten 60 miteinander verbunden werden. Da die Stimulationseinheiten 70 jedoch im Gegensatz zu den Stimulationseinheiten 60 keine Stecker aufweisen, werden zwei benachbarte Stimulationseinheiten 70 mittels eines Steckverbinders, der in die jeweiligen Buchsen 71 der beiden Stimulationseinheiten 70 eingreift, elektrisch und mechanisch miteinander verbunden.

Im Folgenden werden Thermoreize, die mit den in dieser Anmeldung beschriebenen Stimulationseinheiten erzeugt werden können, beschrieben. Derartige Thermoreize lassen sich auch der deutschen Patentanmeldung Nr. 10 2010 000 390.5 mit dem Titel "Vorrichtung und Verfahren zur Behandlung eines Patienten mit Vibrations-, Tast- und/oder Thermoreizen" entnehmen, die am 11. Februar 2010 beim Deutschen Patent- und Markenamt hinterlegt worden ist.

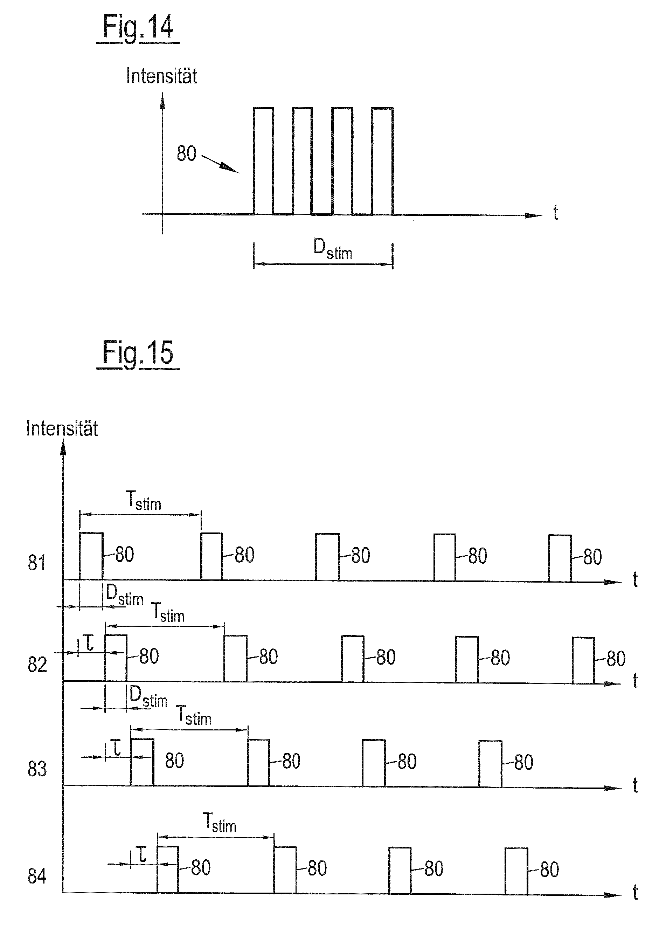

Fig. 14 zeigt einen Thermoreiz 80 mit einer Stimulationsdauer Dₛₜᵢₘ, während der die von der Stimulationseinheit erzeugte Strahlung periodisch mit der Frequenz fthermo = 1/Tₜₕₑᵣₘₒ variiert wird. Die Variation kann z. B. durch An- und Ausschalten der Strahlungsquelle oder durch Modulation der von der Strahlungsquelle konstant emittierten Strahlung erzeugt werden. Durch die Wahl einer geeigneten Wellenlänge, insbesondere Infrarotstrahlung, wird der Bereich der Dermis, in dem sich die Thermorezeptoren befinden, erwärmt.

Die Stimulationsdauer Dₛₜᵢₘ des Thermoreizes 80 kann im Bereich von 10 bis 2000 ms liegen. Die Frequenz fₜₕₑᵣₘₒ kann zwischen 0,01 und 10 Hz oder auch außerhalb dieses Bereichs liegen. Durch den Thermoreiz 80 wird in dem stimulierten Bereich der Dermis eine Temperatur von bis zu 42 °C erzeugt.

Anstelle von pulsförmigen Thermoreizen können auch anders ausgestaltete Thermoreize, z. B. zeitlich kontinuierliche Reizmuster wie etwa Sinusreize verwendet werden. Die Frequenz der Sinusschwingungen kann im Bereich von 0,01 bis 150 Hz und insbesondere im Bereich von 60 bis 150 Hz liegen.

Die von der Stimulationseinheit applizierten Thermoreize werden von Thermorezeptoren aufgenommen und an das Nervensystem weitergeleitet. Zu den Thermorezeptoren zählen Warmrezeptoren (auch Wärmerezeptoren, Warmsensoren oder Wärmesensoren genannt) und Kaltrezeptoren (auch Kälterezeptoren, Kaltsensoren oder Kältesensoren genannt).

Die hier beschriebene Thermostimulation kann insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Tourette-Syndrom, Autismus, Funktionsstörungen nach Schlaganfall, Funktionsstörungen bei Zustand nach Hirntrauma, Spastik, Tinnitus, Schlafstörungen, Schizophrenie, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerzen, allgemeine Kopfschmerzen, Spannungskopfschmerzen, neuropathische Schmerzen, chronische Schmerzzustände, Neuralgie, Amputationsschmerzen, Ataxie, Tic-Störung oder Hypertonie, sowie weiteren Erkrankungen, die durch krankhaft gesteigerte neuronale Synchronisation gekennzeichnet sind. Es können aber auch gastrointestinale Erkrankungen, wie z. B. das Reizdarm-Syndrom, behandelt werden. Hierbei können schmerzhafte Verkrampfungen und ineffiziente Darmmotilität verlernt werden. Auch bei der Colitis ulcerosa und beim Morbus Crohn kann die Thermostimulation krampflösend und schmerzlindernd wirken. Des Weiteren können Asthma bronchiale, COPD (chronisch obstruktive Lungenerkrankungen), kardiale Ischämien sowie die periphere arterielle Verschlusskrankheit behandelt werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

Bei der hier beschriebenen Thermostimulation werden die dem Patienten verabreichten Thermoreize von den Thermorezeptoren aufgenommen und von dort über das Nervensystem an eine krankhaft aktive Neuronenpopulation im Gehirn und/oder Rückenmark weitergeleitet. Die Thermoreize sind so ausgestaltet, dass die krankhaft synchrone Aktivität der Neuronenpopulation desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die gezielte Stimulation bestimmter Bereiche des Gehirns oder Rückenmarks wird durch die somatotope Zuordnung von Körperregionen zu diesen Bereichen ermöglicht. Beispielsweise können die Stimulationselemente am Fuß, Unterschenkel und Oberschenkel oder aber an der Hand, dem Unterarm und Oberarm des Patienten angebracht werden. Aufgrund der somatotopischen Gliederung der Nervenleitungsbahnen werden durch die an den jeweiligen Stellen applizierten Reize unterschiedliche Neuronen stimuliert. Die somatotope Zuordnung von Hautstellen zu Bereichen des Gehirns ist beispielsweise in A. Benninghoff et al.: "Lehrbuch der Anatomie des Menschen. Dargestellt unter Bevorzugung funktioneller Zusammenhänge. 3. Bd. Nervensystem, Haut und Sinnesorgane", Urban und Schwarzenberg, München 1964, beschrieben.

Durch die Verwendung mehrerer und an unterschiedlichen Stellen auf der Haut platzierten Stimulationseinheiten können demnach unterschiedliche Bereiche des Gehirns oder Rückenmarks separat stimuliert werden, indem die applizierten Thermoreize über Nervenleitungen an unterschiedliche Zielgebiete, die im Gehirn und/oder Rückenmark liegen, weitergeleitet werden. Die Zielgebiete können während der Thermostimulation mit eventuell unterschiedlichen und/oder zeitversetzten Reizen stimuliert werden.

Bei einer Variante der Thermostimulation, der "Coordinated Reset" (CR)-Stimulation, die sich durch große therapeutische Wirksamkeit und Sicherheit auszeichnet (vgl. z. B. "A model of desynchronizing deep brain stimulation with a demand-controlled coordinated reset of neural subpopulations" von P. A. Tass, erschienen in Biol. Cybern. 89, 2003, Seiten 81 bis 88), werden einer Neuronenpopulation, die eine krankhaft synchrone und oszillatorische Aktivität aufweist, Thermoreize verabreicht, welche in der Neuronenpopulation ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation mittels einer gezielten Stimulation kontrolliert. Da es ferner möglich ist, die krankhafte Neuronenpopulation an unterschiedlichen Stellen zu stimulieren, kann die Phase der neuronalen Aktivität der krankhaften Neuronenpopulation an den unterschiedlichen Stimulationsstellen zu unterschiedlichen Zeitpunkten zurückgesetzt werden. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb einer der Subpopulationen sind die Neuronen nach dem Zurücksetzen der Phase weiterhin synchron und feuern auch weiterhin mit derselben pathologischen Frequenz, aber jede der Subpopulationen weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Thermoreize nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation in Subpopulationen mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

In Fig. 15 ist eine mit vier Stimulationseinheiten 81 bis 84 durchgeführte CR-Stimulation schematisch dargestellt. Mit Hilfe der Stimulationseinheiten 81 bis 84 werden an unterschiedlichen Stellen der Haut des Patienten die jeweiligen Thermorezeptoren mit den in Fig. 14 dargestellten Thermoreizen 80 stimuliert.

Bei der in Fig. 15 dargestellten Ausgestaltung appliziert jede der Stimulationseinheiten 81 bis 84 einen Thermoreiz 80 periodisch mit der Frequenz fₛₜᵢₘ = 1/Tₛₜᵢₘ. Die Frequenz fₛₜᵢₘ kann im Bereich von 0,1 bis 60 Hz und insbesondere im Bereich von 30 bis 60 Hz oder im Bereich von 1 bis 30 Hz oder im Bereich von 1 bis 20 Hz oder im Bereich von 5 bis 20 Hz liegen, kann aber auch kleinere oder größere Werte annehmen. Insbesondere kann die Frequenz fₛₜᵢₘ nahe der mittleren Frequenz der krankhaft rhythmischen Aktivität des Ziel-Netzwerks liegen.

Die Verabreichung der Thermoreize 80 über unterschiedliche Stimulationseinheiten 81 bis 84 erfolgt mit einer zeitlichen Verzögerung τ zwischen den einzelnen Stimulationseinheiten 81 bis 84 um Tₛₜᵢₘ/4.

Im Fall von N Stimulationseinheiten kann die zeitliche Verzögerung τ zwischen jeweils zwei aufeinander folgenden Thermoreizen 80 beispielsweise im Bereich eines N-tels der Periode 1/fₛₜᵢₘ liegen, d. h. 1 / (N x fₛₜᵢₘ) = Tₛₜᵢₘ/N. In diesem Fall vergeht zwischen den Startzeitpunkten von zwei aufeinander folgenden Thermoreizen 80 folglich die Zeit Tₛₜᵢₘ/N. Von der Vorgabe, dass die zeitliche Verzögerung τ zwischen jeweils zwei aufeinander folgenden Thermoreizen Tₛₜᵢₘ/N beträgt, kann bis zu einem gewissen Grad abgewichen werden. Beispielsweise kann von dem Wert Tₛₜᵢₘ/N für die zeitliche Verzögerung τ um bis zu ± 5%, ± 10% oder ± 20% abgewichen werden. Bei derartigen Abweichungen wurden noch Stimulationserfolge erzielt, d. h., es konnte noch ein desynchronisierender Effekt beobachtet werden.

Die von den Stimulationseinheiten 81 bis 84 applizierten Thermoreize 80 werden an unterschiedliche Subpopulationen der krankhaft synchronen Neuronenpopulation weitergeleitet und resetten die Phasen dieser Subpopulationen zu jeweils unterschiedlichen Zeitpunkten, wodurch eine Desynchronisation der gesamten Neuronenpopulation erzielt wird.

Für die Therapie können unterschiedliche Arten der CR-Stimulation verwandt werden. Eine Möglichkeit besteht in einer "N aus N"-CR-Stimulation, d. h., pro Stimulationszyklus Tₛₜᵢₘ werden wie in Fig. 15 (für N = 4) von allen N Stimulationseinheiten Thermoreize 80 appliziert. Alternativ kann auch eine "L aus N"-CR-Stimulation (mit L < N) durchgeführt werden, bei der pro Stimulationszyklus Tₛₜᵢₘ aus N Stimulationseinheiten L z. B. randomisiert selektiert werden und von diesen die Thermoreize 80 appliziert werden. Auf diese Weise kann eine größere räumliche Variabilität erzeugt werden.

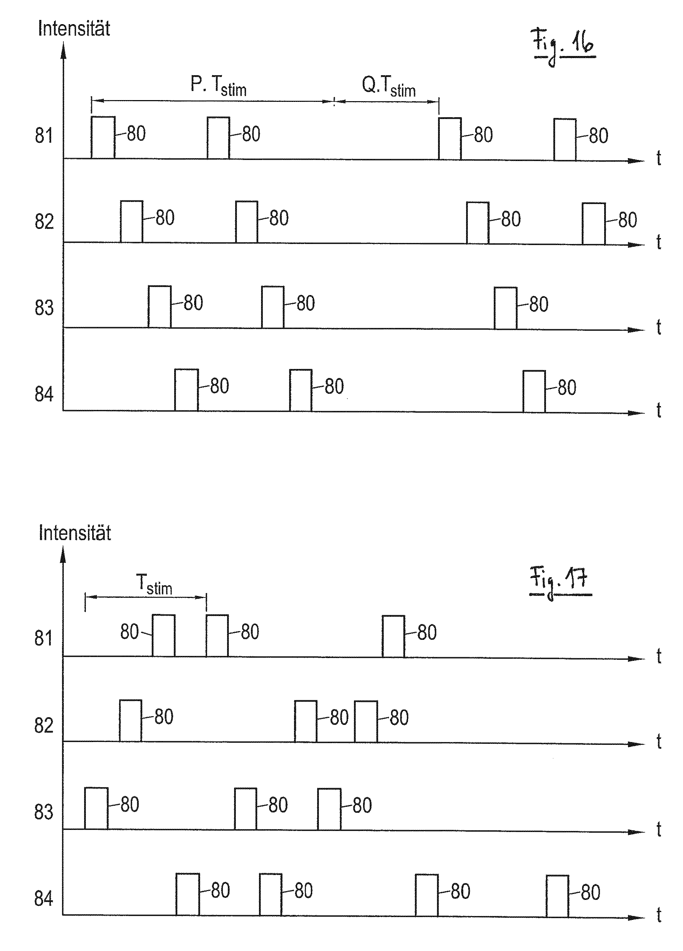

Weitere Variationen der CR-Stimulation mit vier Stimulationseinheiten (N = 4) sind in den Fig. 16 und 17 gezeigt.

Fig. 16 zeigt eine Pause, die während der Applikation der Thermoreize 80 vorgesehen werden kann und während der keine Stimulation erfolgt. Derartige Pausen können beliebig lang gewählt werden und insbesondere ein ganzzahliges Vielfaches der Periode Tₛₜᵢₘ betragen. Ferner können die Pausen nach einer beliebigen Anzahl von Stimulationen eingehalten werden. Z. B. kann eine Stimulation während P aufeinander folgender Perioden der Länge Tₛₜᵢₘ durchgeführt werden und anschließend eine Pause während Q Perioden der Länge Tₛₜᵢₘ ohne Stimulation eingehalten werden, wobei P und Q kleine ganze Zahlen sind, z. B. im Bereich von 1 bis 20. Dieses Schema kann entweder periodisch fortgesetzt werden oder stochastisch und/oder deterministisch, z. B. chaotisch, modifiziert werden.

Eine weitere Möglichkeit, von dem in Fig. 15 gezeigten streng periodischen Stimulationsmuster abzuweichen, besteht darin, die zeitliche Abfolge der Thermoreize 80 stochastisch oder deterministisch oder gemischt stochastisch-deterministisch zu variieren. Fig. 17 zeigt, dass die Reihenfolge, in welcher die einzelnen Stimulationseinheiten die Thermoreize 80 applizieren, pro Periode Tₛₜᵢₘ (oder auch in anderen Zeitschritten) variiert wird. Diese Variation kann stochastisch oder deterministisch oder gemischt stochastisch-deterministisch erfolgen.

Die in Fig. 17 gezeigte Randomisierung kann mit der in Fig. 16 gezeigten Stimulationsform kombiniert werden. Beispielsweise kann in jedem der P aufeinander folgenden Stimulationszeitabschnitte der Länge Tₛₜᵢₘ eine erneute Randomisierung durchgeführt werden oder aber es erfolgt nach jeder Pause der Länge Q x Tₛₜᵢm eine Randomisierung und innerhalb der darauf folgenden P Stimulationszeitabschnitte bleibt die Reihenfolge, in welcher die Stimulationseinheiten die Thermoreize 80 applizieren, konstant.

Des Weiteren kann von dem in Fig. 15 gezeigten streng periodischen Stimulationsmuster abgewichen werden, indem die zeitliche Verzögerung zwischen zwei aufeinander folgenden Thermoreizen 80 nicht stets gleich groß ist. Es kann vorgesehen sein, dass die zeitlichen Abstände zwischen den Thermoreizen 80 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

Der durch die in dieser Anmeldung beschriebene Applikation von Thermoreizen erzielte Stimulationseffekt kann mit Hilfe von Sensoren, welche die neuronale Aktivität in dem stimulierten Zielgebiet oder einem mit dem Zielgebiet verbundenen Gebiet messen, kontrolliert werden. Als Sensoren können nicht-invasive Sensoren eingesetzt werden, z. B. Elektroenzephalographie (EEG)-Elektroden, Magnetenzephalographie (MEG)-Sensoren, Sensoren zur Messung lokaler Feldpotentiale (LFP) und Elektrokardiogramm-Sensoren (EKG). Die neuronale Aktivität kann auch indirekt durch Messung der damit einhergehenden Muskelaktivität mittels Elektromyographie (EMG) ermittelt werden.

Alternativ können die Sensoren in den Körper des Patienten implantiert sein. Als invasive Sensoren können beispielsweise epikortikale Elektroden, Tiefenhirnelektroden, sub- oder epidurale Hirnelektroden, subkutane EEG-Elektroden und sub- oder epidurale Rückenmarkselektroden dienen. Des Weiteren können an peripheren Nerven zu befestigende Elektroden als Sensoren eingesetzt werden.

Mit Hilfe der Sensoren kann ferner die mittlere Frequenz der krankhaft rhythmischen Aktivität des Ziel-Netzwerks gemessen werden. Die Frequenz fₛₜᵢₘ der CR-Stimulation kann an die gemessene Frequenz angepasst werden. Bevorzugt erfolgt diese Frequenzanpassung automatisch, dadurch dass die Vorrichtung über Mittel zur Signalanalyse der von den Sensoren gemessenen Messsignale verfügt. Auf diese Weise kann z. B. aus dem EKG die Herzratenvariabilität (HRV) bestimmt werden. Durch Extraktion der charakteristischen Rhythmen der HRV können durch die Vorrichtung gezielt physiologische vegetative Rhythmen gestärkt bzw. pathologische Rhythmen desynchronisiert werden.

## Patentansprüche

1. Vorrichtung zur Stimulation von in der Haut eines Patienten gelegenen Thermorezeptoren mit Thermoreizen, wobei
- die Vorrichtung mehrere nicht-invasive Stimulationseinheiten (60) zur Bestrahlung der Haut des Patienten mit elektromagnetischer Strahlung und eine Steuereinheit aufweist,
- durch Absorption der elektromagnetischen Strahlung in der Haut des Patienten Thermoreize erzeugt werden,
- die Stimulationseinheiten (60) jeweils eine Hauptoberfläche, an welcher die elektromagnetische Strahlung emittiert wird, und mehrere Seitenflächen, an denen erste und zweite Verbindungselemente (61, 62) vorgesehen sind, aufweisen,
- die Stimulationseinheiten (60) über die ersten und zweiten Verbindungselemente (61, 62) miteinander verbindbar sind, und
- mittels der ersten und zweiten Verbindungselemente (60, 61) eine mechanische Verbindung der Stimulationseinheiten (60) herstellbar ist,
**dadurch gekennzeichnet,dass**
- mittels der ersten und zweiten Verbindungselemente (60, 61) eine elektrische Verbindung der Stimulationseinheiten (60) herstellbar ist,
- die Stimulationseinheiten (60) jeweils an genau einer Seitenfläche ein erstes Verbindungselement (61) und an allen übrigen Seitenflächen ein zweites Verbindungselement (62) aufweisen und wobei die ersten und zweiten Verbindungselemente (61, 62) derart ausgestaltet sind, dass sie ineinander eingreifen und dadurch eine Verbindung herstellen, und
- jede Stimulationseinheit (60) über die zwischen die jeweilige Stimulationseinheit (60) und die Steuereinheit geschalteten Stimulationseinheiten (60) elektrisch mit der Steuereinheit verbunden ist.

2. Vorrichtung nach Anspruch 1, wobei das erste Verbindungselement ein Stecker (61) ist und das zweite Verbindungselement eine Buchse (62) ist.

3. Vorrichtung nach Anspruch 2, wobei Abstandshalter (64) vorgesehen sind, die jeweils an einer Seitenfläche einen Stecker (61) aufweisen, der mechanisch mit einer Buchse (62) einer der Stimulationseinheiten (60) verbindbar ist, wobei die Abstandshalter (64) einen definierten Abstand zwischen den Unterseiten der Stimulationseinheiten (60) und der Haut des Patienten schaffen.

4. Vorrichtung nach einem der vorhergehenden Ansprüche, wobei ein von den Seitenflächen gebildetes Umfangsprofil jeder der Stimulationseinheiten (60) die Form eines regelmäßigen Polygons, insbesondere eines Rechtecks oder eines Quadrats, hat.

5. Vorrichtung nach einem der vorhergehenden Ansprüche, wobei mindestens zwei der Stimulationseinheiten (60) geeignet sind, elektromagnetische Strahlung mit zumindest teilweise unterschiedlichen Wellenlängen zu erzeugen, wobei insbesondere die Stimulationseinheiten (60) in Abhängigkeit von einer gewünschten Eindringtiefe der elektromagnetischen Strahlung in die Haut des Patienten wählbar sind.

6. Vorrichtung nach einem der vorhergehenden Ansprüche, wobei die Stimulationseinheiten (60) jeweils mehrere Strahlung streuende Platten (72) aufweisen und in eine Aussparung jeder Platte (72) jeweils eine Leuchtdiode (73) integriert ist, wobei insbesondere die Flächen der Platten (72), die auf die Haut des Patienten gerichtet sind, für die von den Leuchtdioden (73) erzeugte elektromagnetische Strahlung durchlässig sind und alle anderen Flächen der Platten (72) für die von den Leuchtdioden (73) erzeugte elektromagnetische Strahlung reflektierend sind.

## Claims

1. An apparatus for the stimulation of thermal-receptors lying in the skin of a patient with thermal stimuli, wherein
- the apparatus has a plurality of non-invasive stimulation units (60) for the irradiation of the skin of the patient with electromagnetic radiation and a control unit;
- thermal stimuli are generated in the skin of the patient by means of absorption of the electromagnetic radiation;
- the stimulation units (60) respectively have a main surface at which the electromagnetic radiation is emitted and a plurality of side surfaces at which first and second connection elements (61, 62) are provided;
- the stimulation units (60) can be connected to one another via the first and second connection elements (61, 62); and
- a mechanical connection of the stimulation units (60) can be produced by means of the first and second connection elements (60, 61),
**characterized in that**
- an electrical connection of the stimulation units (60) can be produced by means of the first and second connection elements (60, 61);
- the stimulation units (60) respectively have a first connection element (61) at precisely one side surface and a second connection element (62) at all remaining side surfaces, and wherein the first and second connection elements (61, 62) are configured in such a way that they engage within one another and thereby produce a connection; and
- each stimulation unit (60) is electrically connected to the control unit via the stimulation units (60) interconnected between the respective stimulation unit (60) and the control unit.

2. An apparatus in accordance with claim 1, wherein the first connection element is a plug (61) and the second connection element is a socket (62).

3. An apparatus in accordance with claim 2, wherein spacers (64) are provided which respectively have a plug (61) at a side surface which can mechanically be connected to a socket (62) of one of the stimulation units (60), wherein the spacers (64) create a defined spacing between the lower sides of the stimulation units (60) and the skin of the patient.

4. An apparatus in accordance with any one of the preceding claims, wherein a peripheral profile of each of the stimulation units (60) formed by the side surfaces has the shape of a regular polygon, in particular of a rectangle or of a square.

5. An apparatus in accordance with any one of the preceding claims, wherein at least two of the stimulation units (60) are suitable for generating electromagnetic radiation with at least partly different wavelengths and wherein the stimulation units (60) can in particular be selected in dependence on a desired penetration depth of the electromagnetic radiation into the skin of the patient.

6. An apparatus in accordance with any one of the preceding claims, wherein the stimulation units (60) respectively have a plurality of plates (72) scattering the radiation and a respective light emitting diode (73) is integrated into a cut-out of each plate (72) and wherein the surfaces of the plates (72) which are disposed facing the skin of the patient are in particular transparent with respect to the electromagnetic radiation generated by the light emitting diodes (72) and all other surfaces of the plates (72) are configured reflecting with respect to the electromagnetic radiation generated by the light emitting diodes (73).

## Revendications

1. Dispositif de stimulation de thermorécepteurs situés dans la peau d'un patient, par thermoexcitation, dans lequel
- le dispositif présente plusieurs unités de stimulation (60) non invasives pour irradier la peau du patient avec du rayonnement électromagnétique et une unité de commande,
- des thermoexcitations sont engendrées par absorption du rayonnement électromagnétique dans la peau du patient,
- les unités de stimulation (60) présentent chacune une face principale sur laquelle le rayonnement électromagnétique est émis et plusieurs faces latérales sur lesquelles sont prévus des premiers et deuxièmes éléments de liaison (61, 62),
- les unités de stimulation (60) peuvent être reliées les unes aux autres via les premiers et deuxièmes éléments de liaison (61, 62), et
- au moyen des premiers et deuxièmes éléments de liaison (61, 62) peut être établie une liaison mécanique des unités de stimulation (60), **caractérisé en ce que**
- au moyen des premiers et deuxièmes éléments de liaison (61, 62) peut être établie une liaison électrique des unités de stimulation (60),
- les unités de stimulation (60) présentent chacune sur exactement une face latérale un premier élément de liaison (61) et sur toutes les autres faces latérales un deuxième élément de liaison (62), et les premiers et deuxièmes éléments de liaison (61, 62) sont réalisés de telle sorte qu'ils s'engagent les uns dans les autres et établissent ainsi une liaison, et
- chaque unité de stimulation (60) est reliée électriquement avec l'unité de commande via les unités de stimulation (60) branchées entre l'unité de stimulation (60) respective et l'unité de commande.

2. Dispositif selon la revendication 1, dans lequel le premier élément de liaison est une prise mâle (61 ) et le deuxième élément de liaison est une prise femelle (62).

3. Dispositif selon la revendication 2, dans lequel sont prévus des espaceurs (64) qui présentent chacun sur une face latérale une prise mâle (61) qui peut être reliée mécaniquement avec une prise femelle (62) d'une des unités de stimulation (60), les espaceurs (64) créant une distance définie entre les faces inférieures des unités de stimulation (60) et la peau du patient.

4. Dispositif selon l'une des revendications précédentes, dans lequel un profil de périphérie, formé par les faces latérales, de chacune des unités de stimulation (60), a la forme d'un polygone régulier, en particulier d'un rectangle ou d'un carré.

5. Dispositif selon l'une des revendications précédentes, dans lequel au moins deux des unités de stimulation (60) sont aptes à engendrer du rayonnement électromagnétique avec des longueurs d'ondes au moins partiellement différentes, les unités de stimulation (60) pouvant en particulier être choisies en fonction d'une profondeur de pénétration du rayonnement électromagnétique souhaitée dans la peau du patient.

6. Dispositif selon l'une des revendications précédentes, dans lequel les unités de stimulation (60) présentent chacune plusieurs plaques (72) diffusant du rayonnement et une diode électroluminescente (73) respective est intégrée dans un évidement de chaque plaque (72), en particulier les surfaces des plaques (72) qui sont orientées vers la peau du patient, étant transparentes au rayonnement électromagnétique engendré par les diodes électroluminescentes (73) et toutes les autres surfaces des plaques (72) étant réfléchissantes pour le rayonnement électromagnétique engendré par les diodes électroluminescentes (73).
